# EUROPÄISCHE PATENTSCHRIFT

(11) **EP 1 181 097 B1**
(45) Veröffentlichungstag und Bekanntmachung des Hinweises auf die Patenterteilung: **06.11.2002**
(21) Anmeldenummer: 00943811.0
(22) Anmeldetag: 15.06.2000
(51) Int. Cl.: B01J 37/02, C07C 51/25

(54) **VERFAHREN ZUR HERSTELLUNG VON MONOLITHISCHEN OXIDATIONSKATALYSATOREN UND DEREN VERWENDUNG BEI DER GASPHASENOXIDATION VON KOHLENWASSERSTOFFEN**
METHOD OF PRODUCING MONOLITHIC OXIDATION CATALYSTS AND THEIR USE IN GAS PHASE OXIDATION OF CARBOHYDRATES
PROCEDE DE PRODUCTION DE CATALYSEURS D'OXYDATION MONOLITHIQUES, ET UTILISATION DE CEUX-CI POUR L'OXYDATION EN PHASE GAZEUSE D'HYDROCARBURES

(30) Priorität: 08.07.1999 DE 19931902
(43) Veröffentlichungstag der Anmeldung: 27.02.2002
(73) Patentinhaber: Consortium für Elektrochemische Industrie GmbH, 81379 München (DE)
(72) Erfinder: EBERLE, Hans-Jürgen, D-81477 München (DE); HELMER, Olaf, D-81379 München (DE); STOCKSIEFEN, Karl-Heinz, D-53844 Troisdorf/Bergheim (DE); TRINKHAUS, Stefan, D-80339 München (DE); WECKER, Ulrich, D-82547 Eurasburg (DE); ZEITLER, Norbert, D-80339 München (DE)
(74) Vertreter: Rimböck, Karl-Heinz, Dr.
(86) Internationale Anmeldenummer: EP0005519
(87) Internationale Veröffentlichungsnummer: WO01003832

(56) Entgegenhaltungen:
- EP-A- 0 605 142
- EP-A- 0 965 384
- DATABASE WPI Section Ch, Week 198702 Derwent Publications Ltd., London, GB; Class A97, AN 1987-012100 XP002151362 & JP 61 271034 A (NIPPON SHOKUBAI KAGAKU KOGYO CO LTD), 1. Dezember 1986 (1986-12-01)
- DATABASE WPI Section Ch, Week 198719 Derwent Publications Ltd., London, GB; Class H06, AN 1987-132018 XP002151363 & JP 62 071540 A (TOYO KOGYO CO), 2. April 1987 (1987-04-02)

## Beschreibung

Die Erfindung betrifft ein Verfahren zur Herstellung von monolithischen Oxidationskatalysatoren und deren Verwendung bei der Gasphasenoxidation von Kohlenwasserstoffen.

Trägerkatalysatoren für die Gasphasenoxidation von Kohlenwasserstoffen zu den entsprechenden Oxidationsprodukten wie beispielsweise Carbonsäuren, Carbonsäureanhydriden oder Aldehyde, deren katalytische wirksame Oberflächenbeschichtung im wesentlichen aus Titandioxid (TiO₂) und Divanadiumpentoxid (V₂O₅) besteht, sind seit langem bekannt. Ein typisches Anwendungsbeispiel für derartige Katalysatoren ist die Herstellung von Phthalsäureanhydrid, bei der Gemische aus o-Xylol und Luft oder Naphthalin und Luft oder o-Xylol, Naphthalin und Luft in einem sogenannten Röhrenreaktor über einen entsprechenden Katalysator geleitet werden. Die Ableitung der bei dieser stark exothermen Reaktion entstehenden Wärme (Kühlung, isotherme Reaktionsführung) erfolgt üblicherweise über eine Salzschmelze, mit der die Reaktionsrohre umgeben sind.

Die Trägerkatalysatoren bestehen dabei aus einem inerten Trägerkörper, beispielsweise in Ring- oder Kugelform, auf dem die eigentlich katalytisch aktive Masse aufgebracht ist. Die aktive Masse besteht überwiegend aus den Hauptkomponenten TiO₂ in Anatasform und V₂O₅. Zur Verbesserung der Steuerung der Aktivität und Verbesserung der Selektivität werden dabei häufig noch aktivierende oder auch dämpfende Zusätze, beispielsweise Oxide von Elementen der Nebengruppen des Periodensystems oder Alkaliverbindungen, in geringen Mengen als Dotierstoffe (Promotoren) zu der katalytisch aktiven Masse gegeben.

Bei der Herstellung der Trägerkatalysatoren werden im allgemeinen wäßrige Suspensionen oder wäßrige Lösungen von TiO₂ und V₂O₅, vielfach unter Zusatz von Promotoren und gegebenenfalls Bindemittel zur Haftungsverbesserung der Aktivkomponenten auf dem Träger, auf die Trägerkörper aufgesprüht.

Als Trägerkörper werden regelmäßig geformte, mechanisch stabile Körper wie Kugeln, Ringe, Halbringe, Sättel etc., besonders bevorzugt Ringe oder Kugeln verwendet. Die Größe der Trägerkörper wird dabei vorwiegend von der Dimension des Reaktors, vor allem vom Innendurchmesser der Reaktionsrohre bestimmt.

Als Trägermaterial finden beispielsweise Steatit, Duranit, Steingut, Siliciumdioxid, Siliciumcarbid, Aluminate, Metalle, und Metallegierungen Verwendung.

Aus EP-A 744214 (US-A 5792719) ist eine Verfahrensweise zur Katalysatorherstellung bekannt, bei der TiO₂, V₂O₅, SiC und gegebenenfalls Dotierstoffe wie CsCO₃ und (NH₄)₂HPO₄ mehrere Stunden in wäßriger Suspension gerührt werden, und die Suspension anschließend mit einem organischen Binder versetzt wird. Die Suspension wird auf das Trägermaterial aufgesprüht und der Trägerkatalysator getrocknet.

In der Technik ist es dabei üblich, daß die Reaktionsrohre jeweils mit verschiedenen Katalysatoren befüllt sind, die eine unterschiedliche Zusammensetzung ihrer katalytisch aktiven Masse haben. Dies kann beispielsweise in zwei übereinander liegenden Schichten, einer Oberschicht und einer Unterschicht erfolgen. Mit dieser Maßnahme ist es möglich, das Katalysatorsystem im Reaktor in seiner Aktivität dem Reaktionsverlauf entsprechend anzupassen.

Während der Reaktion wird im oberen Teil des Reaktionrohres der größte Anteil an Kohlenwasserstoff umgesetzt. Zwangsläufig treten damit dort auch die höchsten Temperaturen auf. Im unteren Teil des Rohres findet nur noch eine Art Nachreaktion statt. Dort werden restliches o-Xylol/Naphthalin und Zwischenprodukte, beispielsweise o-Tolyaldehyd und Phthalid zu Phthalsäureanhydrid umgesetzt. Weiterhin werden aber auch Nebenprodukte wie beispielsweise Chinone weiteroxidiert.

Durch Alterungsprozesse verlieren alle Katalysatoren mit zunehmender Gebrauchszeit an Aktivität. Dies geschieht vorwiegend in der Hauptreaktionszone, da dort die höchste Temperaturbelastung stattfindet. Die Hauptreaktionszone wandert dabei im Laufe der Katalystorlebenszeit immer tiefer in das Katalysatorbett. Dadurch wird die Länge des verbleibenden Katalysatorbetts zunehmend verkürzt und die Nachreaktion beeinträchtigt. Daraus ergibt sich, daß Zwischenund Nebenprodukte nicht mehr vollständig umgesetzt werden können und die Produktqualität des erzeugten Phthalsäureanhydids verschlechtert sich damit zunehmend. Besonders kritisch wirkt sich dabei ein Alterungsprozeß bei hohen Feedbeladungen aus. Dem Rückgang der Umsetzung und damit der Verschlechterung der Produktqualität kann zwar durch eine Erhöhung der Reaktionstemperatur, beispielsweise mittels der Salzbadtemperatur entgegengewirkt werden, allerdings nur bis zu einer Temperatur von etwa 400°C. Diese Temperatursteigerung ist aber stets mit einem Ausbeuteverlust verbunden.

In DE-A 1793267 (GB-A 1274471) wird ein Verfahren zur Herstellung von Phthalsäureanhydrid beschrieben, bei dem die oxidative Gesamtreaktion verfahrenstechnisch in zwei Teile unterteilt wird. Die Reaktion wird dabei so gesteuert, daß die Reaktionsbedingungen im zweiten Teil, der sogenannten Nachreaktion, deutlich aggressiver als im ersten Teil ausfallen. Dies kann beispielsweise dadurch erreicht werden, daß die Nachreaktion ohne Kühlung, d.h. adiabatisch durchgeführt wird. Diese Nachreaktion kann dabei in einem separaten Reaktor mit anderen Rohrdimensionen oder gar in einem nachgeschalteten Schachtofen erfolgen.

DE-A 2005969 beschreibt ein Verfahren zur Herstellung von Phthalsäureanhydrid, bei dem etwa 80 bis 99% des gesamten Feeds in der Hauptreaktion isotherm, d. h. gekühlt, umgesetzt werden. Die Umsetzung des verbleibenden nicht umgesetzten Feeds erfolgt in einem nachgeschalteten adiabatischen Reaktor. Zusätzlich wird bei der beschriebenen Reaktionsführung das den isothermen Reaktor verlassende Gasgemisch noch weiter abgekühlt, bevor es in den nachgeschalteten adiabatischen Reaktor eintritt. Durch diese Verfahrensvariante soll das gebildete Phthalsäureanhydrid ebenfalls weitgehend frei von Nebenprodukten und ohne Ausbeuteverlust hergestellt werden können. Auch hier wird ein Schachtofen als adiabatischer Reaktor beansprucht.

Aufgrund der bei Wabenkatalysatoren auftretenden laminaren Strömungsverhältnisse haben diese auch bei sehr hohen Gasgeschwindigkeiten nur einen sehr geringen Druckverlust. Nachteilig ist allerdings, daß wegen der formbedingt fehlenden turbulenten Strömung der Wärme- und Massentransport in den Wabenkanälen, und somit die Wärmeableitung, stark reduziert ist. Dieser Umstand macht eine Anwendung von Wabenkatalysatoren als Katalysatorträger für stark exotherme Prozesse in Verbindung mit einer Selektivoxidation nahezu unmöglich. Wabenkatalysatoren haben sich daher technisch nur in der Abgasreinigung bzw. Abgasverbrennung durchgesetzt, wo die gesamten organischen Bestandteile in einer Totaloxidation zu CO₂ umgesetzt werden.

Die Beschichtung von monolithischem Trägermaterial mit einer katalytisch aktiven Masse aus den Hauptbestandteilen TiO₂, V₂O₅ und gegebenenfalls Dotierstoffen nach allgemein bekannten Verfahren, beispielsweise einem Tauchverfahren, erweist sich als nicht pratikabel. Dies liegt darin begründet, daß Beschichtungssuspensionen auf der Basis von kommerziell verfügbarem TiO₂ bereits bei Feststoffkonzentrationen von 30 - 35 Gew.-% eine sehr hohe Viskosität besitzen, und somit das Beschichten der Kanäle eines monolithischen Trägermaterials ohne diese zu verstopfen nahezu unmöglich macht.

Um monolithische Katalysatorträger mit der notwendigen Menge an katalytisch aktiver Masse, beispielsweise 50 - 150 g aktive Masse pro Liter Katalysator zu beschichten, müßte der Beschichtungsprozeß mit einer derart niedrigkonzenztrierten "aktive Masse"-Suspension durchgeführt werden, daß die notwendige Schichtdicke erst nach mehrmaligem Wiederholen des Beschichtungsprozesses ausreichend wäre. Dadurch erhöht sich aber gleichzeitig wiederum das Problem der Verstopfung der Kanäle im Katalysatorträger aufgrund der mehrmaligen Beschichtung. Darüber hinaus ist dies mit einem deutlich größeren Arbeitsaufwand und damit mit höheren Kosten verbunden und somit unwirtschaftlich.

Es bestand daher die Aufgabe, ein einfaches und möglichst einstufiges Verfahren zur Herstellung monolithischer Katalysatoren auf Basis von TiO₂/Metalloxiden bereitzustellen.

Es wurde nun überraschenderweise gefunden, daß die Viskosität von hochkonzentrierten TiO₂-Beschichtungssuspensionen mit einem hohem Feststoffgehalt durch den Zusatz von Tensiden um ein vielfaches verringert werden kann. Gegenstand der Erfindung ist ein Verfahren zur Herstellung von monolithischen Trägerkatalysatoren für die Gasphasenoxidation durch Beschichtung des Katalysatorträgers mittels einer Suspension, dadurch gekennzeichnet, daß diese aus katalytisch aktiver Masse enthaltend eine oder mehrere TiO₂-Sorten und 1-10 Gew.-% eines oder mehrerer Tenside der allgemeinen Formel

RₙYₘX

besteht, wobei R für den (die) hydrophoben Teil(e) des Tensides mit n gleich 1, 2 oder 3; Y für den hydrophilen Teil des Tensides mit m gleich 0, 1, 2 oder 3 und X für die hydrophile Kopfgruppe des Tensides steht.

Die Verringerung der Viskosität von hochkonzentrierten TiO₂-Beschichtungssuspensionen mit einem Feststoffgehalt größer 30 Gew.-% kann durch den Zusatz von 1 bis 10 Gew.-%, bevorzugt bis 5 Gew.-% an Tensiden der allgemeinen Formel RₙYₘX um ein vielfaches verringert werden.

In dieser allgemeinen Formel steht R für einen oder mehrere hydrophobe Teile, beispielsweise Alkyl-, Aryl- und Alkylaryl-Gruppen eines Tensides, wobei n gleich 1, 2 oder 3, vorzugsweise 1 bis 2 betrågt. Y steht dabei für den hydrophilen Teil eines Tensides, wobei m gleich 0, 1, 2 oder 3, vorzugsweise 0 bis 2 beträgt. X steht für die hydrophile Kopfgruppe des Tensides.

Bevorzugt sind Tenside mit Kopfgruppen X aus der Gruppe. enthaltend Phosphate, Phosphonate, Sulfate, Sulfonate und Carboxylate, Dicarboxylate (Malonsäure-Derivate, Bernsteinsäure-, Adipinsäurederivate, Maleinsäurederivate, Phthalsäurederivate) bzw. Polycarboxylate, beispielsweise mit Tensidresten (R,Y) substituierte Polyacrylate, Polymethacrylate oder Polymaleinsäure-Derivate.

Bei diesen kopfgruppen X kann ein Teil der Säurereste teilweise in der sogenannten H-Form als. freie Säuregruppe, in Form eines Ammoniumsalzes oder als Metallsalz vorliegen. Besonders bevorzugt sind freie Säuregruppen, Ammoniumsalze und Erdalkalisalze.

Die Bindung der hydrophilen Gruppe Y kann direkt oder über einen Sauerstoff an das Zentralatom der Kopfgruppe X erfolgen. Bevorzugte Zentralatome sind Kohlenstoff, Phosphor oder Schwefel.

Vorzugsweise werden die hydrophoben Gruppen R über eine hydrophile Gruppe Y an die Kopfgruppe gebunden.

Bevorzugte Ausführungsform des hydrophoben Teils R sind Alkylreste mit längerkettigen Kohlenstoffbausteinen mit 5 bis 30 C-Atomen, vorzugweise 10 bis 20 C-Atomen. Dabei kann es sich bei den Alkylresten um gesättigte oder ungesättigte oder auch verzweigte Kohlenstoffketten handeln. Die Alkylreste können direkt oder über Arylgruppen an den hydrophilen Teil Y oder die Kopfgruppe X, gebunden sein.

Der hydrophile Rest Y besteht im allgemeinen aus polymeren Alkoxyeinheiten, bevorzugt Propoxy-, Ethoxy- oder Methoxy-Einheiten, wobei der Polymerisierungsgrad zwischen 1 und 50 Monomereinheiten, bevorzugt zwischen 5 und 20 Monomereinheiten liegen kann.

Die erfindungsgemäße Beschichtungssuspension kann beispielsweise Tenside mit der allgemeinen Formel RₙYₘX aus der Gruppe enthaltend Calciumalkylarylsulfonate, Ammoniumalkylarylsulfonate, Dodecylbenzolsulfonat-Calciumsalz, Polyethoxydinonylphenyletherposphat, Polyoxoethylenlauryletherphosphat, Polyethoxytridecyletherphosphat, Calciumdodecylbenzolsulfonat, Tridecylphosphatester, ethoxylierte phosphatierte Alkohole, Alkylpolyoxyethylenetherphosphat, Ammoniumnonylphenylethersulfat beinhalten.

Die Tenside können dabei ohne weiteren Tensidzusatz oder mit anderen Tensiden, beispielsweise Alkylphenolethoxylat eingesetzt werden.

Durch den erfindungsgemäßen Zusatz der Tenside zu der Beschichtungssuspension lassen sich niedrigviskose Beschichtungssuspensionen mit hohen Feststoffgehalten an TiO₂ und/oder V₂O₅ herstellen und zum Beschichten von monolithischem Trägermaterial, beispielsweise Waben und Träger mit offenen oder geschlossenen Kreuzkanalstrukturen verwenden. Die Beschichtungssuspensionen können darüberhinaus aber auch andere Zusatzstoffe, beispielsweise SiC enthalten. Die Feststoffgehalte an katalytisch aktiver Masse in derartigen Suspensionen können dabei auf Werte bis zu 50 Gew.-% und darüber eingestellt werden. Mit derartigen hochkonzentrierten Suspensionen lassen sich monolithische und besonders wabenförmige Katalysatorträger ohne Probleme mit einem Auftrag von 50 bis 150 g aktive Masse pro Liter Wabenkatalysator in einem Beschichtungsschritt realisieren.

Suspensionen mit einem Feststoffgehalt an TiO₂ von größer > 35 Gew.-% weisen aufgrund der hohen Viskosität ein stark reduziertes Fließverhalten auf und können dadurch nicht mehr durch enge Kanäle fließen. Auch der Übergang zu größeren Teilchengrößen bringt keinen Erfolg. Durch den Zusatz von einem oder mehreren der beanspruchten Tenside wird das Fließverhalten dabei deutlich verbessert.

Zur Herstellung der Katalysatoren können einheitliche TiO₂-Sorten oder auch Gemische aus verschiedenen TiO₂-Sorten verwendet werden, die jeweils wiederum mit Metalloxiden dotiert bzw. beschichtet sein können. Vorzugsweise enthält die aktive Masse als zusätzliche Komponente V₂O₅.

Die Beschichtung von Waben mit Beschichtungssuspensionen ohne Zusatz von Tensiden kann nur mit Suspensionen mit einen realtiv geringen Feststoffgehalt von etwa 30 Gew.-% problemlos durchgeführt werden. Die dabei erzielbaren Auftragsmengen an aktiver Masse liegen jedoch nur bei etwa 20 g/l Katalysator. Wird der Feststoffgehalt dabei nur geringfügig erhöht, nimmt die Viskosität der Suspension so stark zu, daß die Suspension nicht mehr aus den Wabenkanälen herausfließen kann und es damit zum Verstopfen der Kanäle kommt.

Durch die Verwendung der anspruchsgemäßen Tenside kann die Beschichtung der Waben ohne Probleme selbst mit Suspensionen mit mehr als 50 Gew.-% an aktiver Masse erfolgen. Auftragsmengen von über 100 g Feststoff/l Wabenkatalysator lassen sich bei Verwendung der beanspruchten Tenside ohne Probleme in einem Beschichtungsvorgang realisieren.

Als Trägermaterialen zur erfindungsgemäßen Beschichtung eignen sich beispielsweise Materialien wie Cordierit, Silicate, Siliciumdioxid, Siliciumcarbid, Aluminiumoxid, Aluminate oder Mischungen aus diesen Stoffen und Metalle bzw. Metallegierungen. Die Trägerkörper können auch geschlossene oder offene Kreuzkanalstrukturen aufweisen. Mit der erfindungsgemäßen Suspensionen können Waben mit einer hohen bis sehr hohen Zelldichte ohne die Gefahr der Verstopfung der Kanäle beschichtet werden.
Bevorzugt sind dabei Waben mit einer Zelldichte, d.h. einer Anzahl der Kanäle von 100 bis 400 csi (cells per square inch), besonders bevorzugt 100 bis 200 csi.

Für die Selektivoxidation von o-Xylol/Luftmischungen mit geringen o-Xylolgehalten zu PSA sind monolithische Katalysatoren sehr gut geeignet. Die monolithischen Katalysatoren neigen dabei in keinem Fall zum Durchgehen der Reaktion. Überraschenderweise sind die monolithischen Katalysatoren dem konventionellen Ringkatalysator (bei gleicher Zusammensetzung der aktiven Masse) überlegen.

Besonders vorteilhaft sind die erfindungsgemäß hergestellten Katalysatoren, die einen Gehalt an aktiver Masse von 40 bis 200 g pro Liter Katalysator besitzen. Bei vergleichbarer Temperatur erzielen diese höhere Umsätze, bessere PSA-Selektivitäten und geringere Mengen an Nebenprodukten.

Die erfindungsgemäß hergestellten Wabenkatalysatoren eignen sich bevorzugt als Katalysatoren für eine Nachreaktion eines PSA-Prozessgases enthaltend ein oder mehrere der Edukte o-Xylol und Naphthalin und/oder Zwischenprodukte wie Tolyaldehyd, Phthalid, Naphthochinon etc. Vorteilhaft wird dabei bei niedrigeren Gaseintrittstemperaturen, bezogen auf die Temperatur des Hauptreaktors, gearbeitet. Dabei läßt sich ein Großteil der Unteroxidationsprodukte aus dem Reaktionsgas entfernen bzw. weiter zu PSA umsetzen. Dies erfolgt überraschenderweise auch bei relativ hohen Raumgeschwindigkeiten von 20.000 - 30.000 h⁻¹. Selbst bei relativ hohen Gehalten an Unteroxidationsprodukten in Verbindung mit einer hohen Konzentration an PSA zeigt sich bei der Verwendung der erfindungsgemäßen Katalysatoren kein "Durchgehen" (Runaway) der Reaktion.

Die erfindungsgemäß hergestellten monolithischen Katalysatoren eignen sich besonders zur Herstellung von Phthalsäureanhydrid in einem adiabatischen Reaktor (Nachreaktor) in Kombination mit einem isotherm geführten Reaktor (Hauptreaktor, beispielsweise gefüllt mit Schüttkatalysator).
Vorzugsweise kann der adiabatische Reaktor dabei auch mit einer vorgeschalteten Gaskühlung betrieben werden. Bei einer besonders bevorzugten Ausführungsform wird die vorgeschaltete Gaskühlung und die adiabatische Reaktion in einem gemeinsamen Apparat durchgeführt.
In der Technik üblich ist, daß vor der Produktisolierung das Reaktionsgas in einem Gaskühler abgekühlt wird. Die vorgeschaltete Gaskühlung, die adiabatische Reaktion im monolithischen Katalysysatorbett und eine weitere Abkühlung kann dabei innerhalb oder außerhalb des Reaktors, bzw. eines gemeinsamen Apparats erfolgen.

Anhand der folgenden Beispiele soll die Erfindung näher erläutert werden.

Die folgenden Beispiele zeigen den Einfluß von Tensiden aus der Gruppe der Phosphorsäureester auf die Viskosität von Trägeroxidsuspensionen. Das Fließverhalten der hergestellten Suspensionen wurde in Anlehnung an DIN 53211 mit einem Auslaufbecher bestimmt. Geprüft wurden zwei Sorten von TiO₂ die sich nur in der Teilchengröße voneinander unterschieden. Der mittlere Teilchendurchmesser betrug 0,1 bzw. 0,4 µm. Dieses Meßverfahren wurde gewählt, da damit ein "Herauslaufen" einer Suspension aus den Wabenkanälen entsprechend simuliert werden konnte. Als Auslaufdüse diente eine Düse mit 2 mm Durchmesser entsprechend einer Anströmfläche von 3,14 mm². Zum Vergleich dazu besitzt eine 200 csi-Wabe einen Kanalquerschnitt von 2,3 mm² und eine 100 csi-Wabe einen Kanalquerschnitt von 4,66 mm². War die Suspension in ihrer Viskosität zu hoch, d.h. die Suspension konnte unter diesen Bedingungen nicht durch die Düse laufen, so wurde der Versuch mit einer Düsenöffnung von 4 mm Durchmesser wiederholt. War auch bei den geänderten Bedingungen kein Auslaufen der Suspension meßbar, wurde der Versuch als "nicht meßbar" gewertet.

### Vergleichsbeispiel 1 (30 Gew.-%ige Suspension ohne Tensid):

Aus einer Mischung von 30 g TiO₂ mit einem mittleren Teilchendurchmesser von 0,1 µm und 70 g Wasser wurde eine Suspension hergestellt und diese 2 Stunden gerührt. Das Fließverhalten wurde anschließend im Auslaufbecher gemessen. Bei einem Düsendurchmesser von 2 mm betrug die Auslaufgeschwindigkeit 1,4 ml/sec.

### Vergleichsbeispiel 2 (33 Gew.-%ige Suspension ohne Tensid):

Aus einer Mischung von 33 g TiO₂ mit einem mittleren Teilchendurchmesser von 0,1 µm und 67 g Wasser wurde eine Suspension hergestellt und diese 2 Stunden gerührt. Das Fließverhalten wurde anschließend im Auslaufbecher gemessen. Bei einem Düsendurchmesser von 2 mm konnte kein Auslauf beobachtet werden. Bei einem Düsendurchmesser von 4 mm betrug die Auslaufgeschwindigkeit 6,2 ml/sec.

### Vergleichsbeispiel 3 (22 Gew.-%ige Suspension ohne Tensid):

Aus einer Mischung von 22 g TiO₂mit einem mittleren Teilchendurchmesser von 0,4 µm und 78 g Wasser wurde eine Suspension hergestellt und diese 2 Stunden gerührt. Das Fließverhalten wurde anschließend im Auslaufbecher gemessen. Bei einem Düsendurchmesser von 2 mm betrug die Auslaufgeschwindigkeit 1,7 ml/sec.

### Vergleichsbeispiel 4 (25 Gew.-%ige Suspension ohne Tensid):

Aus einer Mischung von 25 g TiO₂ mit einem mittleren Teilchendurchmesser von 0,4 µm und 75 g Wasser wurde eine Suspension hergestellt und diese 2 Stunden gerührt. Das Fließverhalten wurde anschließend im Auslaufbecher gemessen. Bei einem Düsendurchmesser von 2 mm konnte kein Auslauf beobachtet werden. Bei einem Düsendurchmesser von 4 mm betrug die Auslaufgeschwindigkeit 8,3 ml/sec.

### Beispiel 5 (45 Gew.-%ige Suspension):

Aus einer Mischung von 45 g TiO₂ mit einem mittleren Teilchendurchmesser von 0,1 µm, 55 g Wasser und 3,5 g Alkyl (C₈-C₁₀)polyoxyethylenetherphosphat wurde eine Suspension hergestellt und diese 2 Stunden gerührt. Das Fließverhalten wurde anschließend im Auslaufbecher gemessen. Bei einem Düsendurchmesser von 2 mm betrug die Auslaufgeschwindigkeit 0,9 ml/sec.

### Beispiel 6 (45 Gew.-%ige Suspension):

Aus einer Mischung von 45 g TiO₂ mit einem mittleren Teilchendurchmesser von 0,4 µm, 55 g Wasser und 3,0 g Alkyl(C₈-C₁₀)polyoxyethylenetherphosphat wurde eine Suspension hergestellt und diese 2 Stunden gerührt. Das Fließverhalten wurde anschließend im Auslaufbecher gemessen. Bei einem Düsendurchmesser von 2 mm betrug die Auslaufgeschwindigkeit 2,0 ml/sec.

### Beispiel 7 (45 Gew.-%ige Suspension):

Aus einer Mischung von 45 g TiO₂ mit einem mittleren Teilchendurchmesser von 0,4 µm, 55 g Wasser und 5,0 g Polyethoxydinonylphenyletherphosphat wurde eine Suspension hergestellt und diese 2 Stunden gerührt. Das Fließverhalten wurde anschließend im Auslaufbecher gemessen. Bei einem Düsendurchmesser von 2 mm betrug die Auslaufgeschwindigkeit 1,2 ml/sec.

### Beispiel 8 (45 Gew.-%ige Suspension):

Aus einer Mischung von 45 g TiO₂ mit einem mittleren Teilchendurchmesser von 0,4 µm, 55 g Wasser und 2,0 g eines Gemisches aus Alkylphenolethoxylat/Alkylarylsulfonat-Calcium wurde eine Suspension hergestellt und diese 2 Stunden gerührt. Das Fließverhalten wurde anschließend im Auslaufbecher gemessen. Bei einem Düsendurchmesser von 2 mm betrug die Auslaufgeschwindigkeit 1,3 ml/sec.

### Beispiel 9 (45 Gew.-%ige Suspension):

Aus einer Mischung von 45 g TiO₂ mit einem mittleren Teilchendurchmesser von 0,1 µm, 55 g Wasser und 5,0 g Dodecylbenzolsulfonat-Calciumsalz wurde eine Suspension hergestellt und diese 2 Stunden gerührt. Das Fließverhalten wurde anschließend im Auslaufbecher gemessen. Bei einem Düsendurchmesser von 2 mm betrug die Auslaufgeschwindigkeit 0,5 ml/sec.

### Beispiel 10 (45 Gew.-%ige Suspension):

Aus einer Mischung von 45 g TiO₂ mit einem mittleren Teilchendurchmesser von 0,4 µm, 55 g Wasser und 2,5 g Ammoniumnonylphenylethersulfat wurde eine Suspension hergestellt und diese 2 Stunden gerührt. Das Fließverhalten wurde anschließend im Auslaufbecher gemessen. Bei einem Düsendurchmesser von 2 mm betrug die Auslaufgeschwindigkeit 1,8 ml/sec.

### Vergleichsbeispiel 11:

Aus einer Mischung von 38 g TiO₂ mit einem mittleren Teilchendurchmesser von 0,4 µm, 9,5 g V₂O₅, und 46,8 g Wasser wurde eine Suspension hergestellt und diese 2 Stunden gerührt. Das Fließverhalten wurde anschließend im Auslaufbecher gemessen. Die Auslaufgeschwindigkeit konnte weder mit einer 2 mm noch 4 mm Düsenöffnung gemessen werden, da in beiden Fällen die Suspension in ihrer Viskosität zu hoch war.

### Beispiel 12:

Aus einer Mischung von 38 g TiO₂ mit einem mittleren Teilchendurchmesser von 0,4 µm, 9,5 g V₂O₅, 46,8 g Wasser und 3 g Alkyl(C₈-C₁₀)polyoxyethylenetherphosphat wurde eine Suspension hergestellt und diese 2 Stunden gerührt. Das Fließverhalten wurde anschließend im Auslaufbecher gemessen. Bei einem Düsendurchmesser von 2 mm betrug die Auslaufgeschwindigkeit 1,3 ml/sec.

Die folgenden Beispiele 13 bis 17 zeigen die Herstellung der Katalysatoren durch Beschichtungsversuche auf entsprechende monolithische Trägerkörper.

### Vergleichsbeispiel 13 (ohne Zugabe von Tensid):

354 g TiO₂ (BET ca. 30m²/g) mit einem mittleren Teilchendurchmesser von 0,1 µm, 118 g TiO₂ (BET < 10m²/g) mit einem mittleren Teilchendurchmesser von 0,4 µm, 120 g V₂O₅ und 8,24 g (NH₄)₂HPO₄ wurden in 1400 ml entionisiertem Wasser suspendiert und 18 Stunden gerührt, um eine homogene Verteilung zu erreichen. Der Feststoffgehalt der erhaltenen Suspension betrug 29,6 Gew.-%. Danach wurden 60 g an organischem Binder, ein Copolymeres aus Vinylacetat und Vinyllaurat, in Form einer 50 Gew.-%igen wäßrigen Dispersion zugegeben. In diese Beschichtungssuspension wurde eine monolithische keramische Trägerwabe aus Cordierit mit einer Zelldichte von 200 cpsi und den Abmessungen 7,5 cm * 7,5cm * 15 cm getaucht und nach etwa 1 Minute Verweilzeit aus dem Tauchbad entnommen. Die sich in den Kanälen befindlichen Reste der Suspension wurden mit einem Luftgebläse (bei maximal 130 °C) ausgeblasen. Die vollständige Trocknung des beschichteten Wabenkörpers erfolgte 12 h in einen Trockenschrank bei 130 °C. Die Auftragsmenge an aktiver Masse betrug 20 g/l Katalysator. Der Wabenkörper war gerade noch beschichtbar.

### Vergleichsbeispiel 14 (ohne Zugabe von Tensid):

Zur Herstellung eines Katalysators mit höherer Auftragsmenge an aktiver Masse wurden 354 g TiO₂ (BET ca. 30m²/g) mit einem mittleren Teilchendurchmesser von 0,1 µm, 118 g TiO₂ (BET < 10m²/g) mit einem mittleren Teilchendurchmesser von 0,4 µm, 120 g V₂O₅ und 8,24 g (NH₄)₂HPO₄ in 1220 ml entionisiertem Wasser suspendiert und 18 Stunden gerührt, um eine homogene Verteilung zu erreichen. Der Feststoffgehalt der erhaltenen Suspension betrug 32,7 Gew.-%. Danach wurden 60 g an organischem Binder, ein Copolymeres aus Vinylacetat und Vinyllaurat, in Form einer 50 Gew.-%igen wäßrigen Dispersion zugegeben. Mit dieser Beschichtungssuspension wurde analog zu Beispiel 15 eine monolithische keramische Trägerwabe aus Cordierit mit einer Zelldichte von 200 cpsi und den Abmessungen 7,5 cm * 7,5 cm * 15 cm beschichtet. Die Suspension war bei diesem Beispiel bereits so hochviskos, daß die Kanäle auch nicht unter Zuhilfenahme des Gebläses vollständig von überschüssiger Suspension befreit werden konnten. Ca. 10 % der Kanäle blieben verstopft. Die beschichtete Wabe war als Katalysator nicht brauchbar.

### Vergleichsbeispiel 15 (Beschichtung von Ringen):

Zur Herstellung des ringförmigen Vergleichskatalysators wurden 73,7 g TiO₂ (BET ca. 30m²/g) mit einem mittleren Teilchendurchmesser von 0,1 µm, 24,6 g TiO₂ (BET < 10m²/g> mit einem mittleren Teilchendurchmesser von 0,4 µm, 25 g V₂O₅ und 1,7 g (NH₄)₂HPO₄ in 400 ml entionisiertem Wasser suspendiert und 18 Stunden gerührt, um eine homogene Verteilung zu erreichen. Danach wurden 6,2 g an organischem Binder, ein Copolymeres aus Vinylacetat und Vinyllaurat, in Form einer 50 Gew.-%igen wäßrigen Dispersion zugegeben. Mit dieser Beschichtungssuspension wurden 1225 g Steatitringe (7x7x4 mm) unter Verdampfung des Wasser auf das Trägermaterial vollständig aufgebracht. Die Schichtdicke an aktiver Masse betrug etwa 60 µm.

### Beispiel 16 (erfindungagemäßhergestellter Katalysator 1):

602 g TiO₂ (BET ca. 30m²/g)mit einem mittleren Teilchendurchmesser von 0,1 µm, 200 g TiO₂ (BET < 10m²/g) mit einem mittleren Teilchendurchmesser von 0,4 µm, 204 g V₂O₅ und 70 g Alkyl(C₈-C₁₀)polyoxyethylenetherphosphat wurden in 980 ml entionisiertem Wasser suspendiert und 18 Stunden gerührt, um eine homogene Verteilung zu erreichen. Der Feststoffgehalt der erhaltenen Suspension betrug 51 Gew.-%. Danach wurden 60 g an organischem Binder, ein Copolymeres aus Vinylacetat und Vinyllaurat, in Form einer 50 Gew.-%igen wäßrigen Dispersion zugegeben. Mit dieser Beschichtungssuspension wurde analog zu Beispiel 15 eine monolithische keramische Trägerwabe aus Cordierit mit einer Zelldichte von 200 cpsi und den Abmessungen 7,5 cm * 7,5 cm * 15 cm beschichtet. Durch die viskositätserniedrigende Wirkung des Tensides lief die nicht an der Wand haftende Suspension problemlos und vollständig aus den Kanälen ab. Aufgrund des hohen Feststoffgehaltes der verwendeten Suspension konnte in einem einmaligen Beschichtungsvorgang eine aktive Masse von 115 g/l Katalysator aufgebracht werden. Es wurde kein Kanal der Wabe mit aktiver Masse verstopft. Die Schichtdicke an aktiver Masse betrug etwa 60 µm.

### Beispiel 17 (erfindungsgemäßhergestellter Katalysator 2):

602 g TiO₂ (BET ca. 30m²/g) mit einem mittleren Teilchendurchmesser von 0,1 µm, 220 g V₂O₅ und 120 g Dodecylbenzolsulfonat-Calciumsalz wurden in 885 ml entionisiertem Wasser suspendiert und 18 Stunden gerührt, um eine homogene Verteilung zu erreichen. Der Feststoffgehalt der erhaltenen Suspension betrug 50 Gew.-%. Danach wurden 60 g an organischem Binder, ein Copolymeres aus Vinylacetat und Vinyllaurat, in Form einer 50 Gew.-%igen wäßrigen Dispersion zugegeben. Mit dieser Beschichtungssuspension wurde analog zu Beispiel 15 eine monolithische keramische Trägerwabe aus Cordierit mit einer Zelldichte von 200 cpsi und den Abmessungen 7,5 cm * 7,5 cm * 15 cm beschichtet. Durch die viskositätserniedrigende Wirkung des Tensides lief die nicht an der Wand haftende Suspension problemlos und vollständig aus den Kanälen ab. Aufgrund des hohen Feststoffgehaltes der verwendeten Suspension konnte in einem einmaligen Beschichtungsvorgang eine aktive Masse von 108 g/l Katalysator aufgebracht werden. Es wurde kein Kanal der Wabe mit aktiver Masse verstopft.

### Beispiel 18 (erfindungsgemäßhergestellter Katalysator 3):

Zur Herstellung eines Katalysator auf Basis eines TiO₂ mit einem größerem mittlerem Teilchendurchmesser wurden 785 g TiO₂ (BET ca. 30m²/g) mit einem mittleren Teilchendurchmesser von 0,4 µm, 196 g V₂O₅ und 46 g Alkyl(C₈-C₁₀)polyoxyethylenetherphosphat in 910 ml entionisiertem Wasser suspendiert und 18 Stunden gerührt, um eine homogene Verteilung zu erreichen. Der Feststoffgehalt der erhaltenen Suspension betrug 52 Gew.-%. Danach wurden 60 g an organischem Binder, ein Copolymeres aus Vinylacetat und Vinyllaurat, in Form einer 50 Gew.-%igen wäßrigen Dispersion zugegeben. Mit dieser Beschichtungssuspension wurde analog zu Beispiel 15 eine monolithische keramische Trägerwabe aus Cordierit mit einer Zelldichte von 200 cpsi und den Abmessungen 7,5 cm * 7,5 cm * 15 cm beschichtet. Durch die viskositätserniedrigende Wirkung des Tensides lief die nicht an der Wand haftende Suspension problemlos und vollständig aus den Kanälen ab. Aufgrund des hohen Feststoffgehaltes der verwendeten Suspension konnte in einem einmaligen Beschichtungsvorgang eine aktive Masse von 97 g/l Katalysator aufgebracht werden. Es wurde kein Kanal der Wabe mit aktiver Masse verstopft.

### Beispiel 19 (Oxidation von o-Xylol/Luftgemischen mit niedriger o-Xylol-Konzentration):

Zur Überprüfung der katalytischen Eigenschaft wurden die Katalysatoren am Beispiel der o-Xyloloxidation auf ihre Eignung hin getestet und mit einem konventionellen Katalysator, hergestellt nach dem Stand der Technik (ringförmige Katalysatoren), verglichen. Die verwendete Testapparatur bestand aus einem adiabatisch geführten d. h. ungekühlten, isolierten Reaktor. Der Reaktor war dabei so gestaltet, daß er mit Katalysatorringen, wie auch mit einem erfindungsgemäßen Wabenkatalysator beladen werden konnte. Dem Reaktor vorgeschaltet war ein Luftvorwärmer, mit dem das o-Xylol/-Luftgemisch auf 300 - 360 °C aufgeheizt werden konnte. Die Katalysatoranströmfläche betrug 19,6 cm², die Katalysatorfüllhöhe 10,2 cm. Die Versuche wurden bei einer Raumgeschwindikeit von 20.000 h⁻¹ durchgeführt. Die Konzentration an o-Xylol im Gasgemisch betrug am Reaktoreingang zwischen 500 und 600 mg/Nm³. Die Messung der o-Xylol-Konzentration erfolgte mit Hilfe der Gaschromatographie sowie eines "on-line" geschalteten FID-Detektors. Das den Reaktor verlassende Reaktionsgas wurde in Aceton aufgefangen und die Bestandteile mittels Gaschromatographie quantitativ bestimmt. Der Gehalt an Kohlenmonoxid und Kohlendioxid im Reaktorausgangsgas wurde über Infrarotmessung direkt erfaßt.

Mit der oben beschriebenen Apparatur wurden die Katalysatoren aus dem Vergleichsbeispiel 13 und aus Beispiel 16, sowie ein ringförmiger Katalysator (Beispiel 15) als weiteren Vergleich getestet. Die Zusammensetzung der aktiven Massen aller drei Katalysatoren war dabei konstant.

Die Testergebnisse sind in folgender Tabelle 1 zusammengefaßt.

**Tab. 1:**

| **Katalysatortests mit niedrig konzentrierten o-Xylol/ Luftgemischen** | | | |
|---|---|---|---|
| | Vergleichskatalysator auf Ringen (Beispiel 15) | Wabenkatalysator mit 20 g aktiver Masse/l Katalysator (Beispiel 13) | Erfindungsgernäßhergestellter Wabenkatalysator, mit 112 g aktiver Masse/l Katalysator (Beispiel 16) |
| Temperatur Gaseintritt | 340°C | 340°C | 340°C |
| o-Xylol Umsatz / mol-% | 56 | 82 | 95 |
| PSA-selektivität / mol-% | 41 | 62 | 67 |
| o-Tolylaldehyd / mg/Nm³ | 22 | 42 | 12 |
| Phthalid / mg/Nm³ | 20 | 36 | 14 |

### Beispiel 20 (Eignung der erfindungsgemäß hergestellten monolithischen Katalysatoren am Beispiel der Nachreaktion von PSA-Prozeßgas aus der o-Xyloloxidation in einem Postreaktor):

Die verwendete Testapparatur (Postreaktor) bestand aus einem adiabatischen geführten (gut isolierten) Reaktionsrohr, in dem der erfindungsgemäße monolithsche Katalysator aus Beispiel 18 eingebaut war. Der Postreaktor war einem üblichen PSA-Pilotreaktor (Hauptreaktor) nachgeschaltet. Die Gasleitung zwischen Hauptreaktor und Postreaktor konnte thermostatisiert werden, so daß variable Gaseintrittstemperaturen in dem Postreaktor möglich waren. Vor Eintritt und am Austritt des Postreaktors waren Gasprobenahmestellen installiert. Des weiteren konnte das Reaktionsgas wahlweise nach Austritt aus dem Hauptreaktor oder nach dem Austritt aus dem Postreaktor in einem Kondensator (Desublimator) abgekühlt und das gebildete PSA abgeschieden bzw. isoliert werden. Der PSA-Hauptreaktor war 3,3 m lang und hatte einen Rohrdurchmesser von 25 mm. Der Reaktor wurde mit einem umgewälzten Salzbad (eutektische Schmelze, aus Kaliumnitrat und Natriumnitrit) temperiert. Die eingespeiste Luftmenge betrug stets 4 Nm³/h. Der PSA-Hauptreaktor war mit einem handelsüblichen PSA-Katalysator bestückt, die Katalysatorfüllhöhe betrug 2,8 m. Die Salzbadtemperaturen wurden dabei so gewählt, daß in dem den Reaktor verlassenden Gasgemisch noch vergleichsweise hohe Gehalte an nicht umgesetzen o-Xylol und Unteroxidationsprodukten wie Phthalid und Toluylaldehyd vorhanden waren. Die Beladung an o-Xylol vor Reaktor betrug während der Versuche konstant 70 g/Nm³ Gas. Das Luft/o-Xylol-Gemisch wurde vor Eintritt in den Hauptreaktor auf 180 °C vorgewärmt.

Das den Hauptreaktor verlassende Reaktionsgas wurde mittels Thermostatisierung auf die gewünschte Temperatur gebracht und durch den mit monolithischen Katalysator beladenen Postreaktor geleitet. Die Menge an monolithischem Katalysator war dabei so gewählt, daß eine Raumgeschwindigkeit von 20.000 h⁻¹ resultierte.
Das den Postreaktor verlassende Reaktionsgas wurde anschließend durch einen Desublimator geleitet, um die Reaktionsprodukte PSA, Phthalid etc. abzuscheiden.

Um die Effizienz des Postreaktors bezüglich dem Abbau bzw. Weiteroxidation der Nebenprodukte beurteilen zu können, wurde ein Teil des PSA-Reaktionsgases vor als auch nach dem Postreaktor mittels Gaswäsche in Aceton mit anschließender gaschromatographischer Bestimmung auf o-Xylol, Phthalid und Tolylaldehyd untersucht. Die Gehalte an CO und CO₂ im Reaktionsgas vor und nach dem Postreaktor erfolgten IR-spektroskopisch. Die Bestimmung der PSA-Ausbeute wurde, wie bereits erwähnt, mittels Abscheidung in einem Desublimator bestimmt sowie über eine Massenbilanzierung berechnet.

Die Ergebnisse der Versuche sind in der folgenden Tabelle 2 dargestellt.

**Tab. 2:**

| **Ergebnisse der Oxidationsversuche mit PSA-Reaktiongas** | | | | | |
|---|---|---|---|---|---|
| | Gehalte* vor/ohne Postreaktor | Gehalte* in Gew.-% nach Postreaktor mit Katalysator aus Bsp. 18 bei verschiedenen Gaseintrittstemperaturen | | | |
| | | 360° C | 340° C | 320° C | 300° C |
| Phthalid | 0,48 Gew.-% | 0,005 | 0,014 | 0,041 | 0,101 |
| o-Xylol | 0,25 Gew.-% | 0 | 0,004 | 0,022 | 0,081 |
| p-Benzochinon | 0,04 Gew.-% | 0,012 | 0,015 | 0,019 | 0,022 |
| o-Tolylaldehyd | 0,21 Gew.-% | 0 | 0,004 | 0,011 | 0,026 |
| PSA-Ausbeute | 111,0 Gew.-% | 111,3 | 111,5 | 111,8 | 112,2 |

| | | | | | |
|---|---|---|---|---|---|
| * Bezogen auf Gesamtsumme an organischen Bestandteilen im Reaktionsgas. | | | | | |

Die Ergebnisse in Tabelle 2 zeigen, daß durch den Einsatz der erfindungsgemäßhergestellten monolithischen Katalysatoren in einem nachgeschalteten adiabatischen Reaktor (Postreaktor) im PSA-Prozeß ein Großteil der Unteroxidationsproduke aus dem Reaktionsgas entfernt und weiter zu PSA oxidiert werden können. Überraschenderweise erfolgt dies auch bei relativ hohen Raumgeschwindigkeiten. Vorteilhaft wird dabei bezüglich der Ausbeute bei niedrigen Gaseintrittstemperaturen gearbeitet. Das Optimum der Reaktionsführung ergibt sich aus der Abwägung von Gehalt an Ausbeute zu Nebenprodukten.

### Beispiel 21: (Eignung der erfindungsgemäß hergestellten monolithischen Katalysatoren am Beispiel der Nachreaktion von PSA-Prozeßgas aus der Naphthalinoxidation in einem Postreaktor):

Der Versuch wurde analog Beispiel 20 durchgeführt, mit der Ausnahme, daß der Hauptreaktor Hauptreaktor mit einem handelsüblichen für die Naphthalinoxidation geeignetem PSA-Katalysator bestückt war und mit einem Naphthalin/Luft-Gemisch beschickt wurde. Im Postreaktor wurde der erfindungsgemäße monolithsche Katalysator aus Beispiel 18 eingebaut. Die Salzbadtemperatur im Hauptreaktor wurde auch in diesem Beispiel so gewählt, daß in dem den Reaktor verlassenden Gasgemisch noch vergleichsweise hohe Gehalte an nicht umgesetzen Naphthalin und das Nebenprodukt Naphthochinon vorhanden waren. Die Beladung des Hauptreaktors an Naphthalin betrug während des Versuchs konstant 70 g/Nm³ bei 4 Nm³ Gas/h.

Die Ergebnisse des Versuches sind in der folgenden Tabelle 3 dargestellt.

**Tab. 3:**

| **Ergebnisse der Oxidationsversuche mit PSA-Reaktiongas aus der Naphthalinoxidation.** | | |
|---|---|---|
| | Gehalte* vor/ohne Postreaktor | Gehalte* nach Postreaktor mit Katalysator aus Bsp. 18 bei einer Gaseintrittstemperatur von 350 °C |
| Naphthalin | 0,12 Gew.-% | 0,02 Gew.-% |
| Naphthochinon | 0,54 Gew.-% | 0,06 Gew.-% |
| PSA-Ausbeute | 99,7 Gew.-% | 100,3 Gew.-% |

| | | |
|---|---|---|
| * Bezogen auf Gesamtsumme an organischen Bestandteilen im Reaktionsgas. | | |

Die Ergebnisse in Tabelle 3 zeigen, daß durch den Einsatz der erfindungsgemäßen monolithischen Katalysatoren in einem nachgeschalteten adiabatischen Reaktor (Postreaktor) im PSA-Prozeß mit Naphthalin als Feed ein Großteil des restlichen Naphthalins und des Nebenproduktes Naphthochinon aus dem Reaktionsgas entfernt und weiter zu PSA oxidiert werden können.

## Patentansprüche

1. Verfahren zur Herstellung von monolithischen Trägerkatalysatoren für die Gasphasenoxidation durch Beschichtung des Katalysatorträgers mittels einer Suspension, **dadurch gekennzeichnet, daß** diese aus katalytisch aktiver Masse enthaltend eine oder mehrere TiO₂-Sorten und 1-10 Gew.-% eines oder mehrerer Tenside der allgemeinen Formel
RₙYₘX
besteht, wobei R für den (die) hydrophoben Teil(e) des Tensides mit n gleich 1, 2 oder 3; Y für den hydrophilen Teil des Tensides mit m gleich 0, 1, 2 oder 3 und X für die hydrophile Kopfgruppe des Tensides steht.

2. Verfahren nach Anspruch 1, **dadurch gekennzeichnet, daß** der Gehalt an Tensid zwischen 2 und 5 Gew.-% liegt.

3. Verfahren nach Anspruch 1 bis 2, **dadurch gekennzeichnet, daß** die Tenside als Kopfgruppe X funktionelle Gruppen aus der Gruppe umfassend Carboxylate, Polycarboxylate, Phosphate, Phosphonate, Sulfate und Sulfonate enthalten.

4. Verfahren nach Anspruch 3, **dadurch gekennzeichnet, daß** die funktionellen Gruppen der Kopfgruppe X ganz oder teilweise als freie Säuregruppe, Ammoniumsalze oder Erdalkalisalze vorliegen.

5. Verfahren nach Anspruch 1 bis 4, **dadurch gekennzeichnet, daß** die Bindung der hydrophilen Gruppe Y direkt oder über einen Sauerstoff an das Zentralatom der Kopfgruppe X erfolgt.

6. Verfahren nach Anspruch 1 bis 5, **dadurch gekennzeichnet, daß** die hydrophoben Gruppen R der verwendeten Tenside über eine hydrophile Gruppe Y an die Kopfgruppe gebunden sind.

7. Verfahren nach Anspruch 1 bis 6, **dadurch gekennzeichnet, daß** die verwendeten Tenside als hydrophoben Teil R gesättigte, ungesättigte oder verzweigte Alkylreste mit Kohlenstoffbausteinen zwischen 5 und 30 C-Atomen besitzen, die direkt oder über Arylgruppen an den hydrophilen Teil Y oder die Kopfgruppe X, gebunden sind.

8. Verfahren nach Anspruch 1 bis 7, **dadurch gekennzeichnet, daß** die verwendeten Tenside hydrophile Reste Y aus polymeren Alkoxyeinheiten besitzen, wobei der Polymerisierungsgrad zwischen 1 und 50 Monomereinheiten liegt.

9. Verfahren nach Anspruch 1 bis 8, **dadurch gekennzeichnet, daß** ein oder mehrere Tenside aus der Gruppe enthaltend Calciumalkylarylsulfonate, Alkylphenolethoxylat, Ammoniumalkylarylsulfonate, Dodecylbenzolsulfonat-Calciumsalz, Polyethoxydinonylphenyletherposphat, Polyoxoethylenlauryletherphosphat, Polyethoxytridecyletherphosphat, calciumdodecylbenzolsulfonat, Tridecylphosphatester, ethoxylierte phosphatierte Alkohole, Alkylpolyoxyethylenetherphosphat, Ammoniumnonylphenylethersulfat verwendet werden.

10. Verfahren nach Anspruch 1 bis 9, **dadurch gekennzeichnet, daß** die katalytisch aktive Masse als zusätzliche Komponente V₂O₅ enthält.

11. Verfahren nach Anspruch 1 bis 10, **dadurch gekennzeichnet, daß** die katalytisch aktive Masse Promotoren enthält.

12. Verfahren nach Anspruch 1 bis 11, **dadurch gekennzeichnet, daß** als Katalysatorträger ein oder mehrere Materialien aus der Gruppe umfassend Cordierit, Silicate, Siliciumdioxid, Siliciumcarbid, Aluminiumoxid, Aluminate, Metalle oder Metallegierungen verwendet werden.

13. Verfahren nach Anspruch 1 bis 12, **dadurch gekennzeichnet, daß** als Katalysatorträgerkörper Waben oder Träger mit offenen und/oder geschlossenen Kreuzkanalstrukturen verwendet werden.

14. Verfahren nach Anspruch 13, **dadurch gekennzeichnet, daß** als Katalysatorträgerkörper Waben mit einer Zelldichte, d.h. einer Anzahl der Kanäle von 100 bis 400 csi (cells per square inch)verwendet werden.

15. Verwendung von monolithischen Trägerkatalysatoren erhältlich nach dem Verfahren gemäß Anspruch 1 bis 14 in einem adiabatischen Reaktor in Kombination mit einem isotherm geführten Reaktor zur Herstellung von Phthalsäureanhydrid aus o-Xylol.

16. Verwendung von monolithischen Trägerkatalysatoren erhältlich nach dem Verfahren gemäß Anspruch 1 bis 14 in einem adiabatischen Reaktor in Kombination mit einem isotherm geführten Reaktor zur Herstellung von Phthalsäureanhydrid aus Naphthalin.

17. Verwendung von monolithischen Trägerkatalysatoren erhältlich nach dem Verfahren gemäß Anspruch 1 bis 14 in einem adiabatischen Reaktor in Kombination mit einem isotherm geführten Reaktor zur Herstellung von Phthalsäureanhydrid aus o-Xylol/Naphthalingemischen

18. Verwendung von monolithischen Trägerkatalysatoren nach Anspruch 15 bis 17 in einem adiabatischen Reaktor mit einer vorgeschalteten Gaskühlung in Kombination mit einem isotherm geführten Reaktor.

19. Verwendung von monolithischen Trägerkatalysatoren nach Anspruch 15 bis 18 in einem adiabatischen Reaktor mit einer vorgeschalteten Gaskühlung, wobei Gaskühlung und Reaktion in einem gemeinsamen Apparat durchgeführt werden, in Kombination mit einem isotherm geführten Reaktor.

20. Verwendung von monolithischen Trägerkatalysatoren nach Anspruch 15 bis 19 in einem adiabatischen Reaktor mit einer vorgeschalteten und einer nachgeschalteten Gaskühlung, wobei Gaskühlung und Reaktion in einem gemeinsamen Apparat durchgeführt werden, in Kombination mit einem isotherm geführten Reaktor.

## Claims

1. Process for producing monolithic supported catalysts for gas-phase oxidation by coating the catalyst support by means of a suspension, **characterized in that** the latter comprises catalytically active composition comprising one or more types of TiO₂ and 1-10% by weight of one or more surfactants of the formula
RₙYₘX
where R is the hydrophobic part(s) of the surfactant and n is 1, 2 or 3; Y is the hydrophilic part of the surfactant and m is 0, 1, 2 or 3, and X is the hydrophilic head group of the surfactant.

2. Process according to Claim 1, **characterized in that** the surfactant content is from 2 to 5% by weight.

3. Process according to Claim 1 or 2, **characterized in that** the head groups X present in the surfactants are functional groups selected from among carboxylates, polycarboxylates, phosphates, phosphonates, sulphates and sulphonates.

4. Process according to Claim 3, **characterized in that** all or some of the functional groups of the head group X are present as free acid groups, ammonium salts or alkaline earth metal salts.

5. Process according to any of Claims 1 to 4, **characterized in that** the hydrophilic group Y is bound to the central atom of the head group X either directly or via an oxygen.

6. Process according to any of Claims 1 to 5, **characterized in that** the hydrophobic groups R of the surfactants used are bound to the head group via a hydrophilic group Y.

7. Process according to any of Claims 1 to 6, **characterized in that** the hydrophobic parts R of the surfactants used are saturated, unsaturated or branched alkyl radicals with carbon building blocks having from 5 to 30 carbon atoms and are bound either directly or via aryl groups to the hydrophilic part Y or the head group X.

8. Process according to any of Claims 1 to 7, **characterized in that** the hydrophilic radicals Y of the surfactants used comprise polymeric alkoxy units whose degree of polymerization is from 1 to 50 monomer units.

9. Process according to any of Claims 1 to 8, **characterized in that** one or more surfactants selected from the group consisting of calcium alkylarylsulphonates, alkylphenol ethoxylates, ammonium alkylarylsulphonates, calcium dodecylbenzenesulphonate, polyethoxy(dinonyl phenyl ether phosphate), polyoxoethylene(lauryl ether phosphate), polyethoxy(tridecyl ether phosphate), calcium dodecylbenzenesulphonate, tridecyl phosphate esters, ethoxylated phosphated alcohols, alkyl polyoxyethylene ether phosphate, ammonium nonyl phenyl ether sulphate, are used.

10. Process according to any of Claims 1 to 9, **characterized in that** the catalytically active composition further comprises V₂O₅ as additional component.

11. Process according to any of Claims 1 to 10, **characterized in that** the catalytically active composition comprises promoters.

12. Process according to any of Claims 1 to 11, **characterized in that** the catalyst supports used are one or more materials selected from the group consisting of cordierite, silicates, silicon dioxide, silicon carbide, aluminium oxide, aluminates, metals or metal alloys.

13. Process according to any of Claims 1 to 12, **characterized in that** the catalyst support bodies used are honeycombs or supports having open and/or closed cross-channel structures.

14. Process according to Claim 13, **characterized in that** the catalyst support bodies used are honeycombs having a cell density, i.e. a number of channels, of from 100 to 400 csi (cells per square inch).

15. Use of monolithic supported catalysts obtainable by the process according to any of Claims 1 to 14 in an adiabatic reactor in combination with an isothermally operated reactor for preparing phthalic anhydride from o-xylene.

16. Use of monolithic supported catalysts obtainable by the process according to any of Claims 1 to 14 in an adiabatic reactor in combination with an isothermally operated reactor for preparing phthalic anhydride from naphthalene.

17. Use of monolithic supported catalysts obtainable by the process according to any of Claims 1 to 14 in an adiabatic reactor in combination with an isothermally operated reactor for preparing phthalic anhydride from o-xylene/naphthalene mixtures.

18. Use of monolithic supported catalysts according to any of Claims 15 to 17 in an adiabatic reactor having upstream gas cooling in combination with an isothermally operated reactor.

19. Use of monolithic supported catalysts according to any of Claims 15 to 18 in an adiabatic reactor having upstream gas cooling, where gas cooling and the reaction are carried out in a joint apparatus, in combination with an isothermally operated reactor.

20. Use of monolithic supported catalysts according to any of Claims 15 to 19 in an adiabatic reactor having upstream gas cooling and downstream gas cooling, where gas cooling and the reaction are carried out in a joint apparatus, in combination with an isothermally operated reactor.

## Revendications

1. Procédé pour la préparation de catalyseurs supportés monolithiques pour l'oxydation en phase gazeuse par revêtement du support de catalyseur au moyen d'une suspension, **caractérisé en ce que** celle-ci est constituée d'une masse catalytiquement active, contenant un ou plusieurs types de TiO₂ et 1 à 10% en poids d'un ou de plusieurs agents tensioactifs de formule générale
RₙYₘX
dans laquelle R représente la ou les parties hydrophobes de l'agent tensioactif avec n égal à 1, 2 ou 3 ; Y représente la partie hydrophile de l'agent tensioactif avec m égal à 0, 1, 2 ou 3 et X représente le groupement de tête hydrophile de l'agent tensioactif.

2. Procédé selon la revendication 1, **caractérisé en ce que** la teneur en agent tensioactif est comprise entre 2 et 5% en poids.

3. Procédé selon les revendications 1 à 2, **caractérisé en ce que** les agents tensioactifs contiennent comme groupement de tête X des groupements fonctionnels du groupe constitué des carboxylates, des polycarboxylates, de phosphates, des phosphonates, des sulfates et des sulfonates.

4. Procédé selon la revendication 3, **caractérisé en ce que** les groupements fonctionnels du groupement de tête X se trouvent totalement ou partiellement sous forme d'un groupement acide libre, de sels d'ammonium ou de sels de métal alcalino-terreux.

5. Procédé selon les revendications 1 à 4, **caractérisé en ce que** la liaison du groupement hydrophile Y est réalisée directement ou via un oxygène sur l'atome central du groupement de tête X.

6. Procédé selon les revendications 1 à 5, **caractérisé en ce que** les groupements hydrophobes R des agents tensioactifs utilisés sont liés via un groupement hydrophile Y au groupement de tête.

7. Procédé selon les revendications 1 à 6, **caractérisé en ce que** les agents tensioactifs utilisés présentent comme partie hydrophobe R des radicaux alkyle saturés, insaturés ou ramifiés présentant des éléments carbonés comprenant entre 5 et 30 atomes de carbone, qui sont liés directement ou via des groupements aryle à la partie hydrophile Y ou le groupement de tête X.

8. Procédé selon les revendications 1 à 7, **caractérisé en ce que** les agents tensioactifs utilisés présentent des radicaux hydrophiles Y d'unités alcoxy polymères, le degré de polymérisation étant compris entre 1 et 50 unités monomères.

9. Procédé selon les revendications 1 à 8, **caractérisé en ce qu'**on utilise un ou plusieurs agents tensioactifs du groupe contenant les alkylarylsulfonates de calcium, le phénoléthoxylate d'alkyle, les alkylarylsulfonates d'ammonium, le sel calcique du benzènesulfonate de dodécyle, le poly(étherphosphate d'éthoxydinonylphényle), le poly(étherphosphate d'oxoéthylènelauryle), le poly(étherphosphate d'éthoxytridécyle), le dodécylbenzènesulfonate de calcium, le phosphatester tridécylique, les alcools phosphatés éthoxylés, le polyoxyéthylèneétherphosphate d'alkyle, le nonylphényléthersulfate d'ammonium.

10. Procédé selon les revendications 1 à 9, **caractérisé en ce que** la masse catalytiquement active contient du V₂O₅ comme composant supplémentaire.

11. Procédé selon les revendications 1 à 10, **caractérisé en ce que** la masse catalytiquement active contient des promoteurs.

12. Procédé selon les revendications 1 à 11, **caractérisé en ce qu'**on utilise comme support de catalyseur un ou plusieurs matériaux du groupe contenant la cordiérite, les silicates, le dioxyde de silicium, le carbure de silicium, l'oxyde d'aluminium, les aluminates, les métaux ou les alliages de métaux.

13. Procédé selon les revendications 1 à 12, **caractérisé en ce qu'**on utilise comme corps support de catalyseur des nids d'abeilles ou des supports présentant des structures de canaux en croix, ouvertes et/ou fermées.

14. Procédé selon la revendication 13, **caractérisé en ce qu'**on utilise comme corps support de catalyseur des nids d'abeilles présentant une densité de cellules, c'est-à-dire un nombre de canaux, de 100 à 400 csi (cellules par pouce carré).

15. Utilisation de catalyseurs supportés monolithiques pouvant être obtenus selon le procédé selon les revendications 1 à 14 dans un réacteur adiabatique en combinaison avec un réacteur conduit de manière isotherme pour la préparation d'anhydride de l'acide phtalique à partir d'o-xylène.

16. Utilisation de catalyseurs supportés monolithiques pouvant être obtenus selon le procédé selon les revendications 1 à 14 dans un réacteur adiabatique en combinaison avec un réacteur conduit de manière isotherme pour la préparation d'anhydride de l'acide phtalique à partir de naphtalène.

17. Utilisation de catalyseurs supportés monolithiques pouvant être obtenus selon le procédé selon les revendications 1 à 14 dans un réacteur adiabatique en combinaison avec un réacteur conduit de manière isotherme pour la préparation d'anhydride de l'acide phtalique à partir de mélanges o-xylène/naphtalène.

18. Utilisation de catalyseurs supportés monolithiques selon les revendications 15 à 17 dans un réacteur adiabatique présentant un refroidissement de gaz disposé en amont en combinaison avec un , réacteur conduit de manière isotherme.

19. Utilisation de catalyseurs supportés monolithiques selon les revendications 15 à 18 dans un réacteur adiabatique présentant un refroidissement de gaz disposé en amont, le refroidissement des gaz et la réaction étant réalises dans un appareil commun, en combinaison avec un réacteur conduit de manière isotherme.

20. Utilisation de catalyseurs supportés monolithiques selon les revendications 15 à 19 dans un réacteur adiabatique présentant un refroidissement de gaz disposé en amont et en aval, le refroidissement des gaz et la réaction étant réalisés dans un appareil commun, en combinaison avec un réacteur conduit de manière isotherme.
